# EUROPEAN PATENT APPLICATION

(11) **EP 1 810 977 A2**
(43) Date of publication of application: **25.07.2007**
(21) Application number: 07107250.8
(22) Date of filing: 24.05.2004
(51) Int. Cl.: C07K 1/04, C07K 1/22, C07K 14/55

(54) **Metal chelate complexes immobilized on solid supports for peptide preparation**

(30) Priority: 23.05.2003 US 472724 P; 05.08.2003 EP 03017839; 16.03.2004 EP 04006195
(62) Divisional of application: 04734508.7
(71) Applicant: AplaGen GmbH, 52499 Baesweiler (DE)
(72) Inventor: The designation of the inventor has not yet been filed
(74) Representative: Meyers, Hans-Wilhelm

(57) **Abstract**

Use of an activated solid phase and an anchoring part which is attached to a peptide for solid phase peptide synthesis, wherein the anchoring part is coordinatively and reversibly attached to the activated solid phase.
Furthermore provided is a process for competitively detachment of said anchoring part, for purification and refolding of said peptides. Provided are peptides with an anchoring part for coordinative and reversible attachment of said peptides to an activated solid phase.

## Description

Chemical synthesis of peptides is well established. In principal, two different methods can be distinguished. The synthesis in solution is often very time consuming and therefore not useful for scientific research, whereas the synthesis on a solid support allows a fast optimization of reaction cycles. The protocols available for solid phase peptide synthesis (SPPS) are based on the Merrifield technique (Merrifield, R.B., J. Amer. Chem. Soc. 85, 1963, 2149), to synthesize peptides with defined sequences on an insoluble solid support which has the benefit that separation of soluble reagents can easily be handled by simple filtration. The SPPS has developed extremely fast to a number of variants (summarized here as Merrifield-type synthesis) and consists of the following steps:
1. In a first step, a suitable solid support matrix (resin) is chosen, to which an amino acid derivative is fixed covalently through its C-term. Usually the first C-terminal amino acid of the sequence to be synthesized is anchored to the solid support by the help of a linker. The amino and side chain functions of this first amino acid are protected by protective groups according to the state of the art, usually compatible with Fmoc- or Boc- chemistry.
2. In a second step, the group protecting the N-term of the first amino acid is selectively removed.
3. In a 3^{rd} step, the next amino acid derivative corresponding to the 2^{nd} amino acid in the sequence to be synthesized is coupled to the free terminal amino group of the first amino acid, while themselves having the amino and side chain function protected by the appropriate protecting groups.
4. In the 4^{th} step, the N-term-protecting group is removed from the freshly coupled amino acid derivative and step 3 is repeated for the next amino acid.

Thus, solid phase synthesis of peptides includes a cyclic process by which a defined sequence of amino acids can be built on a solid phase support. At the end of the process, the peptide is released from the solid support e.g. by treatment with trifluoroacetic acid (including different scavengers).

Simultaneously, the protecting groups which might have been present at side chains of the amino acids, thereby protecting imidazole-, mercapto-, carboxy-, amino-, alcohol- or other functionally relevant groups, might be removed. This leads to the final peptide, which often needs to be purified on suitable chromatographic equipment, usually an LC/MS System, which is able to simultaneously separate and analyse the components. These and further steps are considered in depth in standard textbooks on peptide synthesis.

While synthesis of small peptides (up to 30meres) is usually no problem with SPPS techniques, there are limitations with peptides at a size of 40 up to 120 (or even more) amino acids. Their properties correspond much better to the term "small protein" than to the term "large peptide":
a) Peptides of this size usually have not only the primary structure (sequence), but also a secondary structure (e.g. helix, beta-sheet) and a tertiary structure (e.g. leucine-zipper, disulfide bridging of domains) to be constructed. While the various variants of the SPPS technique aim solely at the build up of the primary structure, they do not provide any tool to control the intramolecular formation of secondary and tertiary structures in a suitable way. The lack of control mechanisms on the folding of larger peptides is even aggravated by the fact that the simultaneous detachment of all products from the resin and removal of protective groups leads to very high local product concentrations. Under these conditions not only intramolecular folding occurs. In many cases, the small proteins released from the resin tend to interact with each other instead of intramolecular folding. Similar problems occur, when the lyophilized fractions containing the purified product eluted from the LC-system are reconstituted with solvent.
   Thus, intramolecular folding and intermolecular interactions compete and there is no technique available to control this process adequately. This often results in functionally useless multimolecular aggregates, which do not show any desired biological activity.
b) In case of the synthesis of large peptides, it is preferred to use appropriately modified (protected, partially protected or unprotected) peptide fragments instead of single amino acid residues for each cycle of synthesis. Usually, small fragments are either connected by ligation or fragment condensation techniques in order to minimize the number of synthetic steps. This is due to the fact that the cycles usually do not have quantitative yields and too many cycles often end in an unacceptable decline of yield. Moreover problematic couplings of single amino acids simply can be prevented by coupling a whole fragment instead of a single amino acid. In the case of fragment condensation the problem arises, that rather long coupling times have to be used during each coupling cycle. The low reaction velocity is diffusion-limited and due to the non-diffusion of the fixed peptide chain and suboptimal diffusion behaviour of the fragments in the pores of the resin surrounding. The long reaction times often lead to a considerable degree of racemisation at the C-terminal amino acid of the fragments to be coupled, which defines a need to keep reaction times as short as possible. The best way to overcome this problem is by coupling in solution. The classical SPPS does not provide an approach to bring both reaction partners in solution and reattach them during the next step of the cycle, while repeating this procedure at each step of the synthesis.
c) It is often difficult to control the desired density of starting residues on a polymeric support and the loading of the resin with the first amino acid often goes along with racemization. Though many preloaded resins are commercially available, these do not cover all loadings and the best value for a given peptide synthesis problem often is hard to find.

Metal affinity had so far only rudimentary application in synthesis of peptides, which is documented in the publication of Comely et al. (Journal of the Chemical Society, 2001, 2526-2531). These authors complexed an amino acid with chromium using aromatic electron-pi-systems e.g. as present in the side chain of derivatized phenylalanine. These complexes were produced in solution and the pre-existing chromium complex was then anchored to a solid phase and one synthesis cycle was performed successfully. While the principal applicability of metal complex anchoring to a solid phase was demonstrated, the procedure differs in the following aspects from the invention presented here.

Comely et al. attach the metal ion to the soluble part of the system first and then anchor the complex to a solid phase in the second step, while the invention presented here is based on attachment of metal ions to a solid phase first and then anchors the growing peptide chain to the solid phase. This abolishes one of the disadvantages of the system presented by Comely, which is that in the resulting chromium-containing complex at the solid phase, the coordinative bond between the amino acid side chains and chromium is stronger than the bond between chromium and the solid support. Thus, elution of the complex with competitors elutes the peptide complex always with chromium in stochiometric amounts being attached to the peptide. Neither for analysis and separation nor for intended pharmaceutical use of a peptide this is suitable. The complex formed by the invention presented here is characterized by strong chelation of the metal ion to the solid phase and easily reversible chelation with the peptide chain. This ends up in elution of the peptide without substantial amounts of metal ions being attached to it.

Comely et al. use very unusual complexes formed by aromatic systems, while the invention presented here is using chelation moieties, which coordinatively bind the metal ion via N, P, S or O atoms. These atoms can be part of standard organic groups. Such groups can be designed and optimized for chelation and strength of binding using the whole repertoire of organic chemistry and are not restricted to aromatic systems. The inventive principle presented here offers thus much greater flexibility and adaptability than the system of Comely.

The system presented by Comely is in so far disadvantageous in that it needs inert atmosphere to protect some of the reagents used. Even under these conditions the yield does not exceed 90% per each step, which means that longer peptides than maximally decameres can not be expected to show appropriate yields. The invention presented here and the complex chemistry used for it are compatible with standard fmoc chemistry and do not need special atmosphere or instrumentation. Comely employs a harsh procedure that is slow and destructive to the resin to set the peptide free (48h oxidation of the grounded polymer with air under white light in DCM).

While no other use was made so far from metal complexes in the field of chemical synthesis of peptides, metal affinity is known in the area of recombinant production of biomolecules. However, it is used solely in a chromatographic setting as a tool for purification of biological molecules from crude biological mixtures or biological fluids in a single step. Patents on such strategies using Agarose-derivatives containing Iminodiacetate and Nitrilotriacetate exist (EP-A-253303; US-A-4877830). Beaded Agarose suited for chromatographic purification of recombinant products is commercially available. Moreover, peptides containing metal-affinity side chains were constructed with the intention to use the side-chain fixed transition metal complexes as luminescent labelling reagents for a given peptide (e.g. WO-A-9603651A1; EPA-A-0178450). All these descriptions document the applicability of metal chelate techniques in the analysis and/or purification of biomolecules. However, they do not imply the nature of the invention and its applications as described below.

The technical problem to be solved was to establish a suitable method for solid phase synthesis, purification and refolding/deaggregation of small and large peptides including such with above 120 amino acids. To circumvent problems of the SPPS for large peptides, the peptides should be reversibly attached to the solid support. Another object of the invention was to refold and separate synthesized peptides having undesired misfolded structures and/or intermolecular aggregates. This problems could be solved by the method of claim 1.

Provided is the use of an activated solid phase comprising a solid support, metal chelating ligands covalently bound to the solid support, metal ions Mⁿ⁺ with n = 1 to 3 coordinatively bound to said metal chelating ligands, said activated solid phase providing coordination sites for the coordinative and reversible attachment of an anchoring part of a peptide for solid phase peptide synthesis or peptide purification.

The activated solid phase is referred to as a "metal affinity resin", too.

In a preferred embodiment the peptide is a "growing peptide" and subject to peptide elongation procedures.

Preferably the growing peptide consists of at least one amino acid. In another preferred embodiment mono- or oligomeric amino acids are added to the carboxy- or aminoterminus (C- or N-terminus) to the "growing peptide" in a Merrifield-type sequential reaction schedule, preferably based on fmoc-chemistry. In a further preferred embodiment the mono- or oligomeric amino acids are/or contain natural and/or unnatural amino acids. Furthermore, the appropriately protected amino acid derivatives or oligomeric fragments being attached in each cycle of the Merrifield-type sequential reaction schedule can be chosen freely.

Preferably the solid support is based on silica, glass or cellulose or a polymer selected from the group consisting of polystyrene resins, melamine resins and polyvinyl alcohol-based resins.

Other suitable supports of the present invention are for example polystyrenes having functional entities, wherein said functional entities can be covalently derivatized with suitable metal chelating ligands. Examples for such functional entities are chlorotrityl, amino, heterocyclic nitrogen, carboxy, hydroxy, mercapto and vinyl groups. For the solid phase peptide synthesis free carboxyl groups or other reactive groups of the solid support have to be protected in a manner that they can not interfere with peptide elongation procedures.

In a preferred embodiment the solid support contains ferromagnetic particles.

In a further preferred embodiment separation of liquid and activated solid phase during synthesis cycles is achieved for example by sieving, size-based separation, centrifugation or magnetic particle separation technology. Reactive functional groups can be introduced to the solid support by means of reaction with pre-existing moieties of the solid support or - in the case of polymers - also by copolymerisation with suitably derivatized copolymers.

In a preferred embodiment each metal chelating ligand contains at least one nitrogen, oxygen, phosphor or sulfur atom which is able to establish a coordinative ligand-metal bond.

In a further preferred embodiment each metal chelating ligand contains at least one functional group selected from the group consisting of amino, heterocyclic nitrogen, carboxy, hydroxyl and mercapto.

The metal chelating ligands are either directly or via linker groups covalently bound to the solid support. Suitable linker groups are for example amino, carboxy, methylene, oxy, methylenedioxy, polymethylenedioxy, ethylenedioxy and polyethylenedioxy groups. For the solid phase peptide synthesis free carboxyl groups of the metal chelating ligands have to be protected in a manner that they do not interfere with peptide elongation procedures.

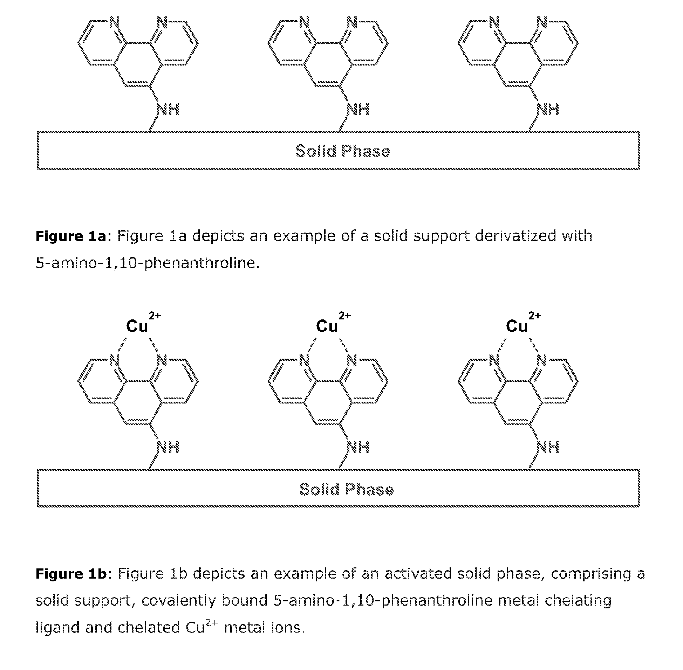

Figure 1a depicts an example of a solid support derivatized with 5-amino-1,10-phenanthroline.

To the skilled person, it is easily possible to identify numerous organic moieties, which are able to coordinatively bind metal ions e.g. from databases which can be used to find appropriate metal chelating moieties for the metal chelating ligands according to this invention. Within the framework of this invention, this existing knowledge can be used to identify suitable moieties, which are in the scope of the invention. An example of a database containing the respective data is given with exemplary data for groups suitable to perform this invention as described herein, given in Table 1, below:

**Table 1: Complex association constants of examplary ligand/metal ion combinations**

| | Association step | **pK** | | | | | |
|---|---|---|---|---|---|---|---|
| Ligand | m | Mn2+ | Fe2+ | Co2+ | Ni2+ | Cu2+ | Zn2+ |
| Imidazole | 1 | 1,25 | 1,81 | 2,44 | 3,01 | 4,21 | 2,55 |
| | 2 | 2,3 | 3,04 | 4,34 | 5,53 | 7,72 | 4,89 |
| | 3 | 3,23 | | 5,76 | 7,5 | 10,57 | 7,17 |
| | 4 | | | 6,7 | 8,8 | 12,6 | 9,18 |
| 1-Methylimidazole | 1 | 1,34 | | 2,29 | 3,05 | 4,22 | 2,38 |
| | 2 | 2,08 | | 4,25 | 5,95 | 7,76 | 4,92 |
| | 3 | 3,08 | | 5,32 | 7,61 | 10,65 | 6,6 |
| | 4 | | | 6,7 | 9,13 | 12,86 | 9,21 |
| Iminodiacetic acid | 1 | 4,72 | 5,8 | 6,97 | 8,3 | 10,56 | 7,15 |
| | 2 | 7,82 | 10,1 | 12,2 | 14,7 | 16,3 | 12,4 |
| | 3 | | | | | | |
| Nitrilotriacetic acid | 1 | 7,27 | 8,9 | 10,38 | 11,51 | 12,7 | 10,45 |
| | 2 | 10,44 | 11,98 | 14,33 | 16,32 | 17,43 | 14,24 |
| Ethylenediaminetetraacetic acid | 1 | 11,1 | 14,3 | 16,5 | 18,4 | 18,8 | 16,5 |
| Ethylenediamine | 1 | 2,6 | 4,13 | 5,5 | 7,3 | 10,49 | 5,69 |
| | 2 | 4,5 | 7,32 | 10,1 | 13,44 | 19,6 | 10,64 |
| | 3 | 5,21 | 9,12 | 13,4 | 17,51 | | 13 |
| 1,3-Diaminopropane | 1 | | | | 6,31 | 9,7 | |
| | 2 | | | | 10,6 | 16,74 | |
| | 3 | | | | 12,9 | | |
| trans-1,2-Diaminocyclohexane | 1 | 2,94 | | 6,37 | 7,9 | 11,09 | 6,37 |
| | 2 | 5,33 | | 11,74 | 14,3 | 20,8 | 11,98 |
| | 3 | | | 15,22 | 20 | | 14,1 |
| 1,4,7-Triazacyclononane | 1 | 5,8 | | 11,2 | 13 | 15,6 | 11,5 |
| | 2 | | | | | 27,7 | |
| 1,4,7,10-Tetraazacyclododecane | 1 | | | 14,1 | 16,4 | 24,6 | 16,2 |
| Pyridin | 1 | 0,24 | 0,7 | 1,22 | 1,88 | 2,54 | 1,05 |
| | 2 | | 0,9 | 1,8 | 3 | 4,38 | 1,45 |
| | 3 | | | | 3,3 | 5,7 | |
| | 4 | | | | | 6,03 | |
| 2,2'-Dipyridyl | 1 | 2,6 | 4,2 | 5,8 | 7,04 | 8,12 | 5,12 |
| | 2 | | 7,9 | 11,3 | 13,86 | 13,63 | 9,63 |
| | 3 | | 17,2 | 16 | 20,16 | 17 | 13,3 |
| 2,2':6',2"-Terpyridine | 1 | 4,44 | 7,1 | 9,5 | 10,7 | 12,3 | 6 |
| | 2 | | 20,7 | 18,6 | 21,8 | 19,1 | |
| 2,4,6-Tri(2-pyridyl)-1,3,5-triazin | 1 | | 12,3 | | | | |
| 4-Hydroxypyridine-2,6- | 1 | | 6,7 | | 8,4 | 9,2 | 12,2 |
| dicarboxylic acid | 2 | | | | 16,2 | 17,3 | 22,1 |
| 1,10-Phenanthroline | 1 | 4,09 | 5,85 | 7,1 | 8,7 | 9,13 | 6,38 |
| | 2 | 7,52 | 11,15 | 13,7 | 16,8 | 15,8 | 12,08 |
| | 3 | 10,2 | 21 | 19,68 | 24,4 | 21,03 | 17,1 |
| 2-Methyl-1,10-phenanthroline | 1 | 3 | 4,2 | 5,1 | 5,95 | 7,4 | 4,96 |
| | 2 | 5,5 | 7,9 | 10 | 11,8 | 13,8 | 9,36 |
| | 3 | 7,9 | 10,8 | 13,9 | 16,7 | 16,95 | 12,7 |

pK values at 25°C. The pK value is definded as the negative decadic logarithm of the complex association constant K. In each column the pK for each consecutive association step of the metal ion or its complex MLₘ₋₁ with an additional ligand molecule L (MeLₘ₋₁ + L ⇄ MLₘ, K=c(MLₘ)/(c(MLₘ₋₁)*c(L)) is shown. Taken from¹.
¹ A.E. Martell, R. M. Smith, R. J. Motekaitis, "Database 46: NIST Critically Selected Stability

Thus, data on the complex formation of different metal ions with ligand molecules are known and can be found in the primary literature and databases. The data in table 1 is taken from the database of Martell and coworkers (2003) and gives an exemplary overview over relevant metal ion ligand interactions. Such databases can be exploited to bind the metal ion to the solid phase. These ligands can also function in the side chain of single or oligomeric natural or unnatural amino acids as a chelating group that binds to the unsaturated metal ion as described below.

In a further preferred embodiment, each metal chelating ligand covalently bound to the solid support contains at least one moiety selected from the group consisting of triphenylphosphine moieties, aminopurine moieties, preferably 6-aminopurine moieties, phthalocyanine moieties, 1,10-phenanthroline moieties, preferably 5-amino-1,10-phenanthroline moieties, terpyridine moieties, preferably 4'-amino-[2,2';6',2"]terpyridine moieties, triazacyclononane moieties, preferably [1,4,7]triazacyclononane moieties and tetraazacyclododecanyl moieties, preferably [1,4,7,10]tetraazacyclododecane moieties.

Preferably the metal Mⁿ⁺ is selected from the group consisting of Mn²⁺, Cu²⁺, Ni²⁺, Co²⁺, Zn²⁺, Mg²⁺, Ca²⁺, Fe²⁺, Fe³⁺ and lanthanide ions, particularly preferred Mⁿ⁺ is Cu²⁺, Ni²⁺, Co²⁺ and _{Zn}²⁺.

Figure 1b depicts an example of an activated solid phase, comprising a solid support, covalently bound 5-amino-1,10-phenanthroline metal chelating ligand and chelated Cu²⁺ metal ions.

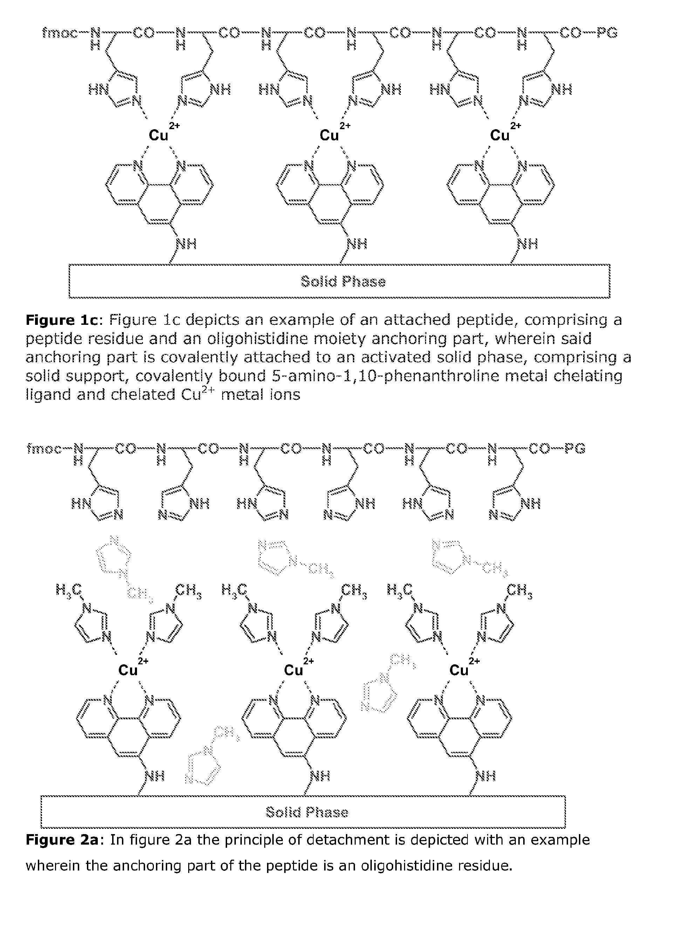

Figure 1c depicts an example of an attached peptide, comprising a peptide residue and an oligohistidine moiety anchoring part, wherein said anchoring part is covalently attached to an activated solid phase, comprising a solid support, covalently bound 5-amino-1,10-phenanthroline metal chelating ligand and chelated Cu²⁺ metal ions.
Constants of Metal Complexes, Version 7", Texas A&M University, College Station, TX

The coverage of the anchored peptides and anchored (mono- or oligomeric) amino acids, the latter one are referred to as a starting point for solid phase peptide synthesis, on the surface of the activated solid support can be controlled very easily. Provided that the coordination sites of the activated solid phase are evenly spread on the surface, the density of attached peptides and starting points (coverage) can be adjusted to the specific needs of synthesis, purification or refolding of peptides and is determined by the amount of starting points added to the resin during the 1^{st} attachment step. The coverage of the surface can be calculated from known surface area values and peptide numbers (measured in moles) and/or diameters.

Since complexation reactions are usually fast, attachment can be completed in the presence of an organic or aqueous solvent within a few minutes.

Provided is a process, wherein an anchoring part of a peptide, said anchoring part is coordinatively bound to coordination sites of an activated solid phase comprising a solid support, metal chelating ligands covalently bound to the solid support, metal ions Mⁿ⁺ with n = 1 to 3 to said metal chelating ligands, is detached from said activated solid phase by addition of a competitive ligand.

According to the invention a competitive agent can be added to the anchored peptides in order to competitively detach the anchored part of the peptide from the activated solid phase. Preferably the competitive agent is added to the reagent mixture of the coupling step of a Merrifield-type reaction schedule. A suitable competitive chelating agent has about the same or weaker affinity for the free coordination sites at the activated solid phase as each individual metal ion chelating moiety of the anchoring part of a peptide to said coordination sites.

In a preferred embodiment the competitive agent is soluble in the reagent mixture of the coupling step and does not react with the ingredients of the reagent mixtures.

In figure 2a the principle of detachment is depicted with an example wherein the anchoring part of the peptide is an oligohistidine residue.

In contrast to the detachment, a reattachment of the anchoring part of the peptide to an activated solid phase is possible by diluting the mixture containing an activated solid phase is, a competitive agent and a non-attached peptide.

In a preferred embodiment within the Merrifield-type reaction schedule, reattachment is carried out prior to the following rinsing steps.

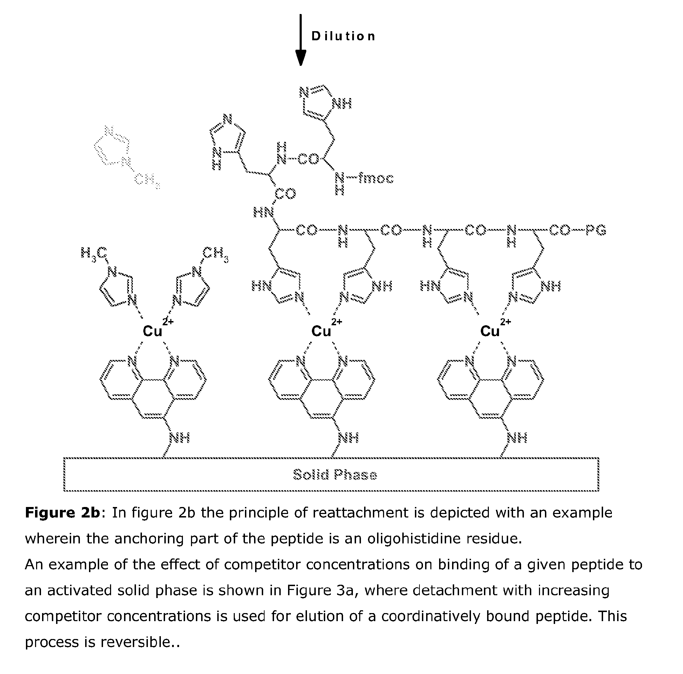

In figure 2b the principle of re-attachment is depicted with an example wherein the anchoring part of the peptide is an oligohistidine residue.

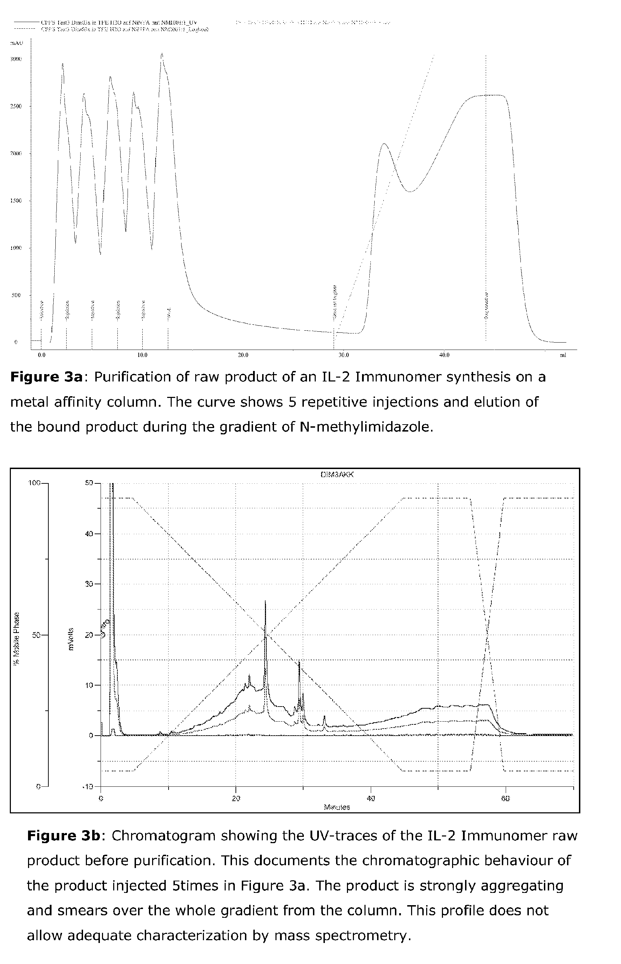

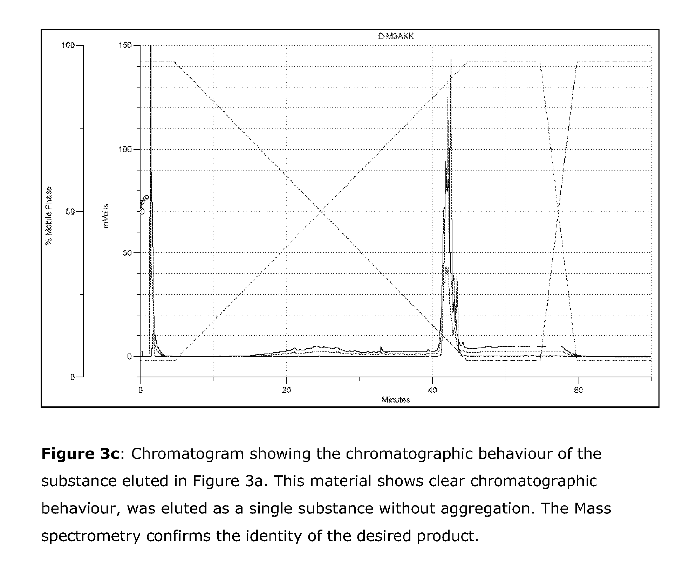

An example of the effect of competitor concentrations on binding of a given peptide to an activated solid phase is shown in Figure 3a, where detachment with increasing competitor concentrations is used for elution of a coordinatively bound peptide. This process is reversible.

In a further provided process, the competitive ligand contains at least one moiety able to chelate metal ions, preferably a nitrogen containing moiety, selected from the group consisting of imidazole, N-methylimidazole, aminopurine, phenanthroline, bipyridine, terpyridine, triazacyclononane and tetraazacyclododecane, iminodiacetic acid moieties, nitrilotriacetic acid moieties and ethylendiaminetetraacetic acid moieties.

In another preferred embodiment, the competitive ligands contains structural moieties having electron pairs for coordinative bonds such as triphenylphosphine moieties, 6-aminopurine moieties or phthalocyanine moieties.

Specific examples for the competitive ligand are glutathione, ethylenediaminotetraacetic acid, imidazole, N-methyl-imidazole, phenanthrolines, preferably 5-amino-1,10-phenanthrolines, aminoterpyridines, triazacyclononanes or tetraazacyclododecanes.

In a preferred embodiment of said process, the peptide is a "growing peptide", bound via an anchoring part to a metal ion, which is bound to a metal chelating ligand bound to a solid support and subject to peptide elongation procedures.

The term "growing" peptide refers to the sequential built up of a peptide backbone, usually using n-terminally and side chain protected amino acids, as e.g. is the case in all fmoc based peptide synthesis protocols.

Preferably mono- or oligomeric amino acids are added at the C- or N-terminus to the "growing peptide" in a Merrifield-type sequential reaction schedule.

As mentioned before, a growing peptide comprising at least one mono- or oligomeric amino acids covalently bound to a metal ion of an anchoring part is regarded as a starting point for solid phase peptide synthesis. Examples for such starting point is a single glycine residue covalently bound via its carboxyl group to a metal ion complexing moiety.

Preferably the starting point comprises at least one side chain and/or C-terminal modification, which is able to coordinate to the metal ions of an activated solid phase. Said amino acids can be natural or unnatural.

Each anchored mono- or oligomeric amino acid used in a starting point for solid phase peptide synthesis, has to be compatible with peptide synthesis protocols.

Preferably the anchoring part of the peptide contains at least one metal ion complexing moiety, each said moiety comprising at least one nitrogen, oxygen, phosphor or sulfur containing group which is able to coordinate to the metal ions of the activated solid phase.

In a preferred embodiment each metal ion complexing moiety contains 1 to 10 of said nitrogen, oxygen, phosphor or sulfur containing groups. More preferred each metal ion complexing moiety contains 1 to 10 amino, heterocyclic nitrogen, aza, carboxy, sulphur and phosphorus containing groups.
The metal ion complexing moieties of the anchoring part can be derivatized, provided that the obtained derivative is compatible with peptide synthesis protocols. Figures 1c, 2a and 2b depict an oligohistidine moiety, which aminoterminus is protected by Fmoc chemistry.

In another embodiment of the invention the coordinative bond of the metal chelating ligands to the metal ions of the activated solid phase is stronger than the coordinative bond of the anchoring part of the peptide to said metal ions.

More preferably the nitrogen containing group, able to coordinate to the metal ions of the activated solid phase, is selected from the group consisting of amino, hydroxyl, carboxyl, mercapto, imidazolyl, N-methylimidazolyl, aminopurinyl moieties, phenanthrolyl moieties, pyridyl moieties, bipyridyl moieties, terpyridinyl moieties, triazacyclononanonyl moieties, tetraazacyclododecanyl moieties, iminodiacetic acid moieties, nitrilotriacetic acid moieties and ethylenediaminetetraacetic acid moieties.

Particularly preferred said moiety comprises 2 to 10 natural or unnatural amino acids. Suitable side chains for said amino acid containing metal ion complexing moieties can preferably be selected from imidazolo, amino, hydroxyl, carboxyl, mercapto, phenanthroline, pyridine, bipyridine, terpyridine, triazacyclononane, tetraazacyclododecane or purine moieties and derivatives thereof, which are still able to form metal complexes. Suitable organic moieties being able to form coordinative bonds with metal ions can be searched in databases as exemplary shown in Table 1. In order to ensure peptide elongation procedures free carboxyl groups have to be suitably protected.

Preferably the mono- or oligomeric amino acids of the anchoring part contain, optionally N-terminally protected, imidazole side chains. In another preferred embodiment said mono- or oligomeric amino acids are oligohistidine or short (1-6 residues) sequences of unnatural amino acids harbouring phenanthroline moieties in their side chains with at least one additional amino acid, wherein the additional amino acid doesn't interfere with the solid support such as glycine.

Preferably said oligohistidine moieties contain at least 2 histidine residues; more preferably 6-10 histidine residues.

In another preferred embodiment said oligohistidine moieties comprise a seqence of at least 2 serial L- or D-histidine residues, more preferably 6 L- or D-histidine residues.

In a further preferred embodiment said mono- or oligomeric amino acids of the anchoring part contain at least one 5-amino-1,10-phenathroline moiety.

It is possible to repetitively detach and reattach the growing peptide chain from and onto the activated solid phase. In a preferred embodiment detachment is achieved during the coupling step of peptide synthesis, while reattachment is induced by dilution of the reaction mixture prior to the rinsing steps preceding the following deprotection. Using these steps, it is possible to use "transient" liquid phase synthesis and its advantages, especially for synthesis of large peptides by fragment condensation approaches.

Preferably said anchoring part of the peptide chain is located at the C-terminus and/or in at least one side chain of the amino acids of the peptide.

In a further preferred embodiment, said anchoring part of the peptide may be extended by specific groups and/or sequences of natural and/or unnatural amino acids wich allows further processing and detecting of the peptides.

In a preferred embodiment at least one amino acid of the anchoring part at the C-terminus of the peptide is extended by one or more amino acids, which allows detection by detection systems.

In a preferred embodiment a sequence is added to the growing peptide, which allows simple recognition of the final peptide chain by an antibody. An example for such tag-sequence is myc-tag. Moreover, addition of a biotinylated amino acid (e.g. biotinylated D-, L-lysine), preferably at the C-terminus of an oligomeric starting point, would allow to establish simple quantification of the final product based on the specific interactions between biotin and avidin-like molecules.

In a further preferred embodiment the anchoring part of the peptide is extended at its N-terminus by an amino acid sequence providing a recognition site for a specific protease.

In a further preferred embodiment the starting point is extended by short amino acid sequences, which code for protease recognition sites. This allows selective removal of the starting point after the synthesis process and the folding protocol.

In a preferred embodiment, the peptide is reattached to the activated solid phase by diluting the reaction mixture of the Merrifield-type sequential reaction schedule containing the competitive ligand.

Suitable competitive ligands have a similar affinity to the activated solid phase (metal affinity resin) compared to the affinity of single residues of the, preferably oligomeric nitrogen groups, of the metal ion complexing moieties of the anchoring part of the peptide (starting point) to be detached. In a case of oligohistidine as anchoring part of the peptide, suitable competitive ligands are imidazole (in aqueous solvents) or N-methyl imidazole (in organic solvents). Detachment is achieved by adding a large excess (typically 10² - 10⁶ molar excess of competitive ligand related to the attached ligand) of competitive ligand compared to the attached peptide to the solvent. For example in aqueous solvents 100 to 250 mM of imidazole (competitive ligand) are suitable to achieve complete detachment. At the same conditions reattachment can be achieved by dilution of the solvent containing the competitive ligand, preferably by a factor of 10 to 20. This leads to a drop in the concentration of the competitive ligand and provides re-attachment of the multidentate anchoring part of the peptide to the support. The molar ratio of the competitive ligand to the anchoring parts can be easily determined for pairs of anchoring part and activated solid phase in the respective solvent by chromatographic procedures eluting the anchoring part from a metal affinity resin.

In a further preferred embodiment attachment of an anchoring part for peptide synthesis (e.g. a oligohistidine) is achieved in the presence of a diluted alkaline or neutral solution of the starting point to an activated solid phase.

Furthermore provided is a process for purification of, optionally protected, peptides containing an anchoring part, said anchoring part is coordinatively bound to coordination sites of an activated solid phase comprising a solid support, metal chelating ligands covalently bound to the solid support, metal ions Mⁿ⁺ with n = 1 to 3 to said metal chelating ligands, is rinsed, to wash away contaminants such as remnants of protecting groups and scavengers or undesired side products of peptide synthesis.

Another preferred embodiment of the invention makes use of chelating groups attached in final steps of the synthesis to a peptide (at the amino-terminal side of the chain, e.g. a Gly-phenanthroline derivative). In this case, purification of the raw product from contaminating side products is achieved by a chromatographic procedure making use of the coordinativly attachment of a peptide to an activated solid phase. This principle can be applied by using regular end-capping of free uncoupled amino groups in each synthesis cycle. In applying this principle, purification of a raw product can be achieved within a single chromatographic run.

Detachment can be achieved by addition of a suitable competition agent as described above or by increasing acidity of the solvent to a critical degree, preferably at least to below pH 6, more preferably to pH 5 or below, especially in aqueous solution, which offers another elegant way of detaching an anchored peptide from a metal affinity resin.

Provided is another process, based on the aforementioned coordinative and reversible attachment of a peptide to an activated solid phase.

A process for refolding misfolded structures and/or deaggregating intermolecular aggregates of an, optionally protected peptide, wherein the anchoring part of the peptide is coordinatively and reversibly attached to an activated solid phase, and re-establishing a correctly folded peptide structure, comprising the steps of
(a) exposure of the peptide to at least one chaotropic or denaturing agent, and
(b) (b) subsequent exposure to a sequence of solvents to gradually reduce chaotropy and to provide reproducible conditions of refolding and re-establishment of secondary and tertiary structure.

In a preferred embodiment the chaotropic or denaturing agent is selected from the group consisting of urea, detergents such as sodium dodecylsulfate, high salt concentrations and mercaptoethanol or mixtures thereof in a suitable solvent.

Instead of covalent attachment to a suitable resin the present invention provides the possibility to detach the growing peptide chain during the coupling step, while it can be attached again before the following steps. Moreover, the same principle of reversible anchoring of a peptide to an activated solid phase can be used to control folding of a purified product. For this purpose, the product is purified and reattached to an activated solid phase, wherein the ratio of attached peptide molecules (measured in moles) to surface area of metal affinity resin is chosen in such a way that attached peptides are not likely to interact with each other. This prefers intramolecular folding instead of intermolecular interactions (aggregation). Having been purified and attached, the product is then treated with a number of solvents which allow a gradual folding of the molecule and support the correct intramolecular configuration of the peptide chains. The folding steps can be sealed by the formation of e.g. disulfide bonds in the correct position. At the end of this procedure the stabilized product is released from the activated solid phase by addition of a competitive ligand or by increasing acidity of the solvent.

Refolding of the attached peptide is carried out in a refolding protocol. For this protocol, the product has to be dissolved in a suitable solvent. Usually, the most preferred solvent is the elution solvent from a LC-chromatography system, although the product can be lyophilized and reconstituted in a suitable solvent. From this solution the product is coordinatively reattached to an activated solid phase. This can be done including the use of resins based on unprotected iminodiacetic acid or trinitriloacetic acids. This is possible since refolding usually does not use reagents, which activate free carboxylic groups. Density of attachment (coverage of the surface) is chosen very low in order to avoid that product molecules come into close contact with each other. After completion of attachment a series of solvents is passed gradually along the resin. In principle the first solvents are highly denaturing and/or chaotropic in order to bring all product molecules in a comparable state of folding. Gradually, the next steps and solvents will approach physiological conditions in such a way that molecular folding is supported. At the end of the protocol, the product molecules are in the desired, preferably aqueous, final solvent, optionally sealed with e.g. a disulfide bond and can be released from the activated solid phase.

In a further preferred embodiment, the secondary and tertiary structure of a peptide is maintained by covalent links between reactive side chains of said peptide by treating the peptide with suitable agents, comprising the formation of said covalent links prior to detachment of the peptide from the activated solid phase.

Preferably the covalent links formed between reactive side chains of the refolded peptides are disulfide bonds, amide bonds or stable aromatic or aliphatic hydrazones.

Suitable agents for closing e.g. disulfide bridges can be selected from various redox reagents such as iodine, ferrocyanates, oxygen and peroxides. Other reagents, which are established for closure of covalent bonds in convention solution phase reactions can be chosen, too.

Provided is a process, wherein the secondary and tertiary structure of correctly folded peptides are fixed by the formation of covalent links between side chains of amino acids, preferably by closure of disulfide bonds from free mercapto groups, the formation of amide bonds, or the formation of stable aromatic or aliphatic hydrazones are achieved by passing reagent mixtures along the refolded product, which is attached to the activated solid phase.

Provided is furthermore a specific process, for the synthesis of of a peptide of the sequence

HHHH-XX-TIVESCNRWITFAQSIISTLT-βAla-G-G-βAla-TKKTQLQLEHLLLDLQMCLNGINN-XX (I)

with, X = d-alanine and βAla = beta-alanine, comprising the formation of a disulfide bond between the cysteine residues, thus forming 
S—S
HHHH-XX-TIVESCNRWITFAQSIISTLT-βAla-G-G-βAla-TKKTQLQLEHLLLDLQMCLNGINN-XX,
 wherein X and βAla are as defined above.

The specific process for the synthesis of the peptide of the sequence (I) which is synthesized on an activated solid phase using the process described above in a Merrifield-type sequential reaction schedule, comprising at least one step of purification and refolding and formation of a disulfide bond between the cysteine residues. The same applies to peptide derivatives thereof with amino acid deletions or amino acid replacements, since still two cyctein residues are present in a distance which allows formation of a disulfide bond.

Furthermore provided are peptides containing an anchoring part preferably consisting of unnatural amino acids for coordinative and reversible attachment of the peptide to the surface of an activated solid phase, which anchoring part is located at the N- or C-terminal and/or in a side chain of the peptide, contains at least one metal ion complexing moiety, each said moiety comprising at least one nitrogen containing group, with the exception of peptides, wherein said metal ion complexing moiety is an amino acid sequence of 2 to 5 histidine residues.

Preferably the metal ion complexing groups, preferably nitrogen containing groups of the provided peptides, are fixed to the N- or C-terminus or part of the side chain of at least one amino acid and are selected from the group consisting of amino, hydroxyl, carboxyl, mercapto, imidazolyl, N-methylimidazolyl, aminopurinyl moieties, phenanthrolyl moieties, pyridyl moieties, bipyridyl moieties, terpyridinyl moieties, triazacyclononanonyl moieties and tetraazacyclododecanyl moieties, or combinations thereof.

In another preferred embodiment the amino acid moiety comprises single or oligomeric natural or unnatural amino acids.

In a preferred embodiment, the anchoring part contains at least one imidazolyl moiety. More preferably, the anchoring part of the peptide contains 1 to 10 imidazolyl moieties.

In another preferred embodiment the amino acid contains at least two, more preferably 6 to 10 histidinyl moieties. In a preferred embodiment the amino acid moiety contains at least one imidazolyl moiety. More preferably, the anchoring part contains 1 to 10 imidazolyl moieties.

Another advantage associated with the invention is that metal-affinity resins can be reused after peptide synthesis. This can be achieved by removing the cation and reloading the resin with the same or another appropriate cation. Since the binding strength of the various cations is different from each other, a method of modifiying the attachment and re-attachment procedures and the stability of the resulting complexes is to choose for another cation as bridging ligand between the resin surface and the starting point of the synthesis.

The invention is further illustrated by the following non-limiting examples.

**Examples:**

| Abbreviations | Substance |
|---|---|
| DMF | N,N-Dimethylformamide (peptide synthesis grade) |
| DCM | Dichloromethane (peptide synthesis grade) |
| HOBT | 1-Hydroxybenzotriazole (anhydrous) |
| DBU | 1,8-Diazabicyclo[5,4,0]undec-7-en 98% |
| PyBOP | Benzotriazole-1-yl-oxy-tris-pyrrolidino-phosphonium-hexafluorophosphate |
| DIPEA, DIEAN-Ethyldiisopropylamin | 98+% |
| TIS | Triisopropylsilan 99% |
| MeOH | Methanol HPLC (gradient grade) |
| TFE | 2,2,2-Trifluorethanol 99.8% |
| EDT | 1,2-Ethanedithiol |
| HCl | Hydrochloric acid |
| NaOH | Sodium hydroxide solution |
| THF | Tetrahydrofurane |
| DMSO-d₆ | Dimethylsulfoxide, deuterated |
| br | broad (signal) |
| s | singlet |
| d | doublet |
| t | triplet |
| q | quartet |
| p | quintet |
| m | multiplet |
| TFA | Trifluoroacetic acid |
| NMI | N-Methylimidazole |
| Ni-NTA | Superflow Resin (Novagen) |

### Example 1: Synthesis of Fmoc-Gly-His₄-Gly-OMe [TAG1]

2mmol of the first amino acid and 4mmol DIPEA are dissolved in 10ml dry DCM. This solution is added to 1.0g of dry 2-chlorotrityl chloride resin (200-400 mesh) and the mixture allowed to react for 60 minutes on a vortexer. At the end of the reaction the resin is allowed to react twice with 20ml of DCM/MeOH/DIPEA for 3 minutes, washed twice with 20ml DCM and twice with DMF. The resin is then treated twice with 20ml piperidine/DMF (1:3) for 3/20 minutes and washed 6 times with 20ml DMF.

5mmol of the amino acid, 7.5mmol HOBT and 10mmol DIPEA are dissolved in 15ml DMF. After 5 minutes 5mmol of PyBOP and 10mmol DIPEA are added and this solution is poured onto the resin. After 60 minutes on a vortexer the resin is washed 6 times with 20ml DMF, treated twice with 20ml piperidine/DMF (1:3) for 20/20 minutes and washed 6 times with 20ml DMF. In case of the N-terminal amino acid the resin is not treated with piperidine/DMF.

After attachment of the last amino acid the resin is washed 6 times with 20ml DMF, twice with 20ml DCM and then allowed to react with 50ml TFE/DCM (2/8) for 60 minutes. The resin is filtered off, the solvents removed in vacuo and the crude peptide fragment used without further purification.

The crude peptide, 10mmol CL-HOBT, 10mmol DIEA and 20mmol DIC are disolved in a minimum dry DCM. The solution is allowed to react for 10 minutes and a solution of 10mmol DIEA and 10mmol Gly-Ome is added. After 2h of vortexing 100ml DCM is added and the solution extracted 3 times with 200ml portions of sodiumhydrogensulfate (1-2mol/l), 3 times with 200ml portions of concentrated sodiumchloride and3 times with 200ml portions of sodiumhydrogencarbonate (1-2mol/l). The organic layer is dried over sodiumsulfate, the solvent removed and the crude peptide used without further purification.

The crude peptide is dissolved in 50ml TFA/TIS/H₂O (95/2.5/2.5) and allowed to react for 60 minutes on a vortexer. TFA is removed by coevaporation with DCM. The crude peptide was dissolved in DMSO and 1000µl purified on a Gilson Nebula LCMS System using a Kromasil RP C18 column. The linear gradient extended from 5% aqueous TFA (0.1%) to 50% acetonitrile (containing 0.085% TFA) over 30 min. The flow rate was 20 mL/min and the absorbance monitored at 214nm.

### Example 2: Synthesis of Fmoc-Gly-His₆-Gly-OMe [TAG3]

2mmol of the first amino acid and 4mmol DIPEA are dissolved in 10ml dry DCM. This solution is added to 1.0g of dry 2-chlorotrityl chloride resin (200-400 mesh) and the mixture allowed to react for 60 minutes on a vortexer. At the end of the reaction the resin is allowed to react twice with 20ml of DCM/MeOH/DIPEA for 3 minutes, washed twice with 20ml DCM and twice with DMF. The resin is then treated twice with 20ml piperidine/DMF (1:3) for 3/20 minutes and washed 6 times with 20ml DMF.

5mmol of the amino acid, 7.5mmol HOBT and 10mmol DIPEA are dissolved in 15ml DMF. After 5 minutes 5mmol of PyBOP and 10mmol DIPEA are added and this solution is poured onto the resin. After 60 minutes on a vortexer the resin is washed 6 times with 20ml DMF, treated twice with 20ml piperidine/DMF (1:3) for 20/20 minutes and washed 6 times with 20ml DMF. In case of the N-terminal amino acid the resin is not treated with piperidine/DMF.

After attachment of the last amino acid the resin is washed 6 times with 20ml DMF, twice with 20ml DCM and then allowed to react with 50ml TFE/DCM (2/8) for 60 minutes. The resin is filtered off, the solvents removed in vacuo and the crude peptide fragment used without further purification.

The crude peptide, 10mmol Cl-HOBT, 10mmol DIEA and 20mmol DIC are disolved in a minimum dry DCM. The solution is allowed to react for 10 minutes and a solution of 10mmol DIEA and 10mmol Gly-Ome is added. After 2h of vortexing 100ml DCM is added and the solution extracted 3 times with 200ml portions of sodiumhydrogensulfate (1-2mol/l), 3 times with 200ml portions of concentrated sodiumchloride and3 times with 200ml portions of sodiumhydrogencarbonate (1-2mol/l). The organic layer is dried over sodiumsulfate, the solvent removed and the crude peptide used without further purification.

The crude peptide is dissolved in 50ml TFA/TIS/H₂O (95/2.5/2.5) and allowed to react for 60 minutes on a vortexer. TFA is removed by coevaporation with DCM. The crude peptide was dissolved in MEOH and 1000µl purified on a Gilson Nebula LCMS System using a Kromasil RP C18 column. The linear gradient extended from 5% aqueous TFA (0.1%) to 80% acetonitrile (containing 0.085% TFA) over 50 min. The flow rate was 20 mL/min and the absorbance monitored at 214nm.

### Example 3: Synthesis of Fmoc-Gly-His₃-Gly-His₃-Gly-OMe [TAG4]

2mmol of the first amino acid and 4mmol DIPEA are dissolved in 10ml dry DCM. This solution is added to 1.0g of dry 2-chlorotrityl chloride resin (200-400 mesh) and the mixture allowed to react for 60 minutes on a vortexer. At the end of the reaction the resin is allowed to react twice with 20ml of DCM/MeOH/DIPEA for 3 minutes, washed twice with 20ml DCM and twice with DMF. The resin is then treated twice with 20ml piperidine/DMF (1:3) for 3/20 minutes and washed 6 times with 20ml DMF.

5mmol of the amino acid, 7.5mmol HOBT and 10mmol DIPEA are dissolved in 15ml DMF. After 5 minutes 5mmol of PyBOP and 10mmol DIPEA are added and this solution is poured onto the resin. After 60 minutes on a vortexer the resin is washed 6 times with 20ml DMF, treated twice with 20ml piperidine/DMF (1:3) for 20/20 minutes and washed 6 times with 20ml DMF. In case of the N-terminal amino acid the resin is not treated with piperidine/DMF.

After attachment of the last amino acid the resin is washed 6 times with 20ml DMF, twice with 20ml DCM and then allowed to react with 50ml TFE/DCM (2/8) for 60 minutes. The resin is filtered off, the solvents removed in vacuo and the crude peptide fragment used without further purification.

The crude peptide, 10mmol CL-HOBT, 10mmol DIEA and 20mmol DIC are disolved in a minimum dry DCM. The solution is allowed to react for 10 minutes and a solution of 10mmol DIEA and 10mmol Gly-Ome is added. After 2h of vortexing 100ml DCM is added and the solution extracted 3 times with 200ml portions of sodiumhydrogensulfate (1-2mol/l), 3 times with 200ml portions of concentrated sodiumchloride and3 times with 200ml portions of sodiumhydrogencarbonate (1-2mol/l). The organic layer is dried over sodiumsulfate, the solvent removed and the crude peptide used without further purification.

The crude peptide is dissolved in 50ml TFA/TIS/H₂O (95/2.5/2.5) and allowed to react for 60 minutes on a vortexer. TFA is removed by coevaporation with DCM. The crude peptide was dissolved in DMSO and 1000µl purified on a Gilson Nebula LCMS System using a Kromasil RP C18 column. The linear gradient extended from 5% aqueous TFA (0.1%) to 50% acetonitrile (containing 0.085% TFA) over 30 min. The flow rate was 20 mL/min and the absorbance monitored at 214nm.

### Example 4: Synthesis of Fmoc-Gly-5-Amino-1,10-phenanthroline [TAG5]

2ml DIEA, 6mmol Cl-HOBt and 12mmol DIC are added to a solution of 2.56mmol Fmoc-Gly-OH in a minimum of DMF. This solution is vortexed for 5 minutes and a solution of 0.5g 5-Amino-1,10-phenantroline in a minimum of DMF is added. The mixture is incubated for 12 hours and diluted with the 5fold volume of ethylacetate. The solution is washed three times with sodium hydrogencarbonate. The crude product precipitates, is filtered off and purified by column chromatography or LCMS. The crude peptide was dissolved in DMSO and 1000µl purified on a Gilson Nebula LCMS System using a Kromasil RP C18 column. The linear gradient extended from 5% aqueous TFA (0.1%) to 50% acetonitrile (containing 0.085% TFA) over 50 min. The flow rate was 20 mL/min and the absorbance monitored at 214nm.

### Example 5: Synthesis of (Bis-tert-butoxycarbonylmethyl-amino)-acetic acid [TAG8b]

### a) Synthesis of (Bis-tert-butoxycarbonylmethyl-amino)-acetic acid benzyl ester [TAG8a]

20 mmol Glycine benzyl ester *p*-tosylate, 40 mmol Bromo-acetic acid tert-butyl ester and 60 mmol DIEA are dissolved in 35 ml dry DMF. The reaction mixture is vortexed for 4 days. Precipitated salts are filtered off and the solution is dissolved in 250 ethyl acetate. The organic layer is extracted with the following solutions: 2x 200 ml 1N NaOH, 3x 1N NaOH/brine 1:1. The solution is dried with Na₂S0₄, the solvent removed by vacuum destillation and the crude product is purified by flash column chromatography (ethyl acetate/hexane 1:4).
¹H-NMR (400 MHz, DMSO-d₆) ppm 7.42-7.29 (m, 5H), 5.10 (s, 2H), 3.60 (s, 2H), 3.50 (s, 4H), 1.38 (s, 18H); LC-MS (ESI): (M+H)⁺ = 394

### b) Synthesis of (Bis-tert-butoxycarbonylmethyl-amino)-acetic acid [TAG8b]

17.5 mmol (Bis-tert-butoxycarbonylmethyl-amino)-acetic acid benzyl ester is dissolved in 75 ml THF and the catalyst palladium, 10% on charcoal, 0.70 g,is added. The reaction vessel is flushed several times with hydrogen and the reaction mixture is stirred under hydrogen until the hydrogen consumption stops. The catalyst is filtered off over celite and the solvent is removed in vacuo.
¹H-NMR (400 MHz, DMSO-d₆) ppm, 12.2 (br s, 1H), 3.46 (s, 2H), 3.44 (s, 4H), 1.40 (s, 18H); LC-MS (ESI): (M+H)⁺ = 304

### Example 6: Synthesis of 4-(Bis-tert-butoxycarbonylmethylcarbamoyl)-butyric acid [TAG10b]

### a) 4-(Bis-tert-butoxycarbonylmethyl-carbamoyl)-butyric acid benzyl ester [TAG10a]

24 mmol Pentanedioic acid monobenzyl ester, 30 mmol oxalyl chloride, and two drops DMF are dissolved in 20 ml dry DCM. The reaction mixture is refluxed until the gas evolution stops. The solution is coevaporated with 10 ml toluene. The crude product is dissolved in DCM and added drop by drop to a solution of 12.0 mmol (tert-Butoxycarbonylmethyl-amino)-acetic acid tert-butyl ester and 26.4 mmol DIEA in DCM at 0°C. The reaction mixture is stirred for 1h at 0°C and at room temperature overnight. The solvent is removed in vacuo and the residue is dissolved in 150 ml diethyl ether. The organic layer is extracted with the following solutions: 100 ml 1N HCI, 100 ml half-concentrated sodium hydrogencarbonate solution, and 100 ml brine. The solution is dried over sodium sulfate and the solvent removed in vacuo. The crude product is purified by silica column chromatography (ethyl acetate/hexane 1:1).
¹H-NMR (400 MHz, DMSO-d₆) ppm 7.4-7.2 (m, 5H), 5.08 (s, 2H), 4.11 (s, 2H), 3.91 (s, 2H), 2.38 (t, J = 7.4 Hz, 2H), 2.28 (t, J = 7.3 Hz, 2H), 1.75 (p, J=7.4 Hz, 2H), 1.41 (s, 9H), 1.39 (s, 9H); LC-MS (ESI): (M+H)⁺ = 450

### b) Synthesis of 4-(Bis-tert-butoxycarbonylmethyl-carbamoyl)-butyric acid [TAG10b]

2 mmol 4-(Bis-tert-butoxycarbonylmethyl-carbamoyl)-butyric acid benzyl ester is dissolved in 20 ml THF and the catalyst palladium, 10% on charcoal, 0.09 g,is added. The reaction vessel is flushed several times with hydrogen and the reaction mixture is stirred under hydrogen until the gas consumption stops.

The catalyst is filtered off over celite and the solvent is removed in vacuo. ¹H-NMR (400 MHz, DMSO-d₆) ppm 12.1 (br s, 1H), 4.11 (s, 2H), 3.91 (s, 2H), 2.30-2.17 (m, 4H), 1.68 (p, J=7.2 Hz, 2H), 1.42 (s, 9H), 1.39 (s, 9H); LC-MS (ESI): (M+Na)⁺ = 382

### Example 7: Synthesis of 4-([1,10]Phenanthrolin-5-ylcarbamoyl)-butyric acid [TAG12b]

### a) Synthesis of 4-([1,10]Phenanthrolin-5-ylcarbamoyl)-butyric acid methyl ester [TAG12a]

In an argon atmosphere 5 mmol 5-amino-[1,10]-phenanthroline are dissolved in 25 ml dry DMF. 6.5 mmol DIEA are added and 5.5 mmol 4-chlorocarbonylbutyric acid methyl ester are added drop by drop. After stirring for 1h the solvent and excess base are removed by vacuum destillation. The crude product is purified by reversed phase column chromatography (Merck Lobar RP18 column, eluant: acetonitrile/ammonium hydrogencarbonate, 5 weight-%, gradient: 20% to 40%)

¹H-NMR (400 MHz, DMSO-d₆) ppm 10.11 (s, 1H), 9.13 (dd, J=1.6, 4.3 Hz, 1H), 9.03 (dd, J=1.7, 4.3 Hz, 1H), 8.60 (dd, J=1.6, 8.4 Hz, 1H), 8.44 (dd, J=1.7, 8.2 Hz, 1H), 8.17 (s, 1H), 7.82 (dd, J=4.2, 8.4 Hz, 1H), 7.74 (dd, J=4.3, 8.1 Hz, 1H), 3.63 (s, 3H), 2.59 (t, J=7.2 Hz, 2H), 2.46 (t, J=7.4 Hz, 2H), 1.95 (p, J=7.3Hz, 2H); LC-MS (ESI): (M+H)⁺ = 324

### b) Synthesis of 4-([1,10]Phenanthrolin-5-ylcarbamoyl)-butyric acid [TAG12b]

1.5 mmol 4-([1,10]Phenanthrolin-5-ylcarbamoyl)-butyric acid methyl ester are dissolved in 15 ml 1,4-dioxane and 15 ml destilled water. 1.5 ml of 1 N solution of potassium hydroxide are added and the solution is refluxed for 1 hour. The solvent is removed by vacuum destillation and the crude product is purified by reversed phase column chromatography (Merck Lobar RP18 column, eluant: acetonitrile/trifluoroacetic acid, 1 weight-%, gradient: 10% to 20%)

¹H-NMR (400 MHz, CD₃OD) as K salt: ppm 9.11 (dd, J=1.6, 4.3 Hz, 1H), 9.04 (dd, J=1.6, 4.4 Hz, 1H), 8.65 (dd, J=1.5, 8.4 Hz, 1H), 8.41 (dd, J=1.6, 8.1 Hz, 1H), 8.14 (s, 1H), 7.82 (dd, J=4.4, 8.4 Hz, 1H), 7.74 (dd, J=4.4, 8.1 Hz, 1H), 2.64 (t, J=7.6 Hz, 2H), 2.36 (t, J=7.2 Hz, 2H), 2.10 (q, J=7.5 Hz, 2H) ); LC-MS (ESI): (M+H)⁺ = 310

Furthermore tags containing other dicarboxylic acids derivatives than glutaric acid can be prepared analogously to the here described protocols.

### Example 8: Synthesis of [5-([1,10]Phenanthrolin-5-ylcarbamoyl)-pentanoylamino]-acetic acid [TAG15]

In an argon atmosphere 1 mmol Wang resin that is loaded with Fmoc-Gly-OH (0.7 mmol/g) is suspended in piperidine/DMF 1:3 and irridiated in a microwave oven (system "Discover" by CEM) three times for 30 seconds. The resin is washed three times with DMF and three times with DCM. The resin is suspended in 15 ml DCM and 9 mmol DIEA and 10 mmol adipoylchloride are added. After vortexing the suspension for 30 minutes, the reaction solution is filtered off and the resin is washed two times with DCM and one time with DMF. The resin is suspended in 15 ml DMF and a solution of 3 mmol 5-amino-1,10-phenanthroline and 5 mmol DIEA in 20 ml DMF is added. After vortexing the suspension overnight the resin is washed six times with DMF and the product cleaved from the resin by suspending in TFA/TIS/H₂O 95:2.5:2.5. The resin is filtered off and the filtrate is coevaporated several times with chloroform to yield the product.

This procedure can also be applied analogously to other resin bound peptides and other activated derivatives of dicarboxylic acids.

### Example 9: 5-Amino-1,10-phenantroline / 2-Chlortritylchloride-resin

2mmol 5-Amino-1,10-phenantroline are suspended under inert atmosphere in 50ml dry DMF. To the resulting mixture 5g 2-Chlorotritylchloride-resin (200-400mesh) is added and the resin is incubated at room temperature for 2hours. The resin is filtered off and washed with DMF until the excess of Phenantroline is removed. To block unreacted groups on the resin, it is incubated three times with 10ml DCM/MeOH/DIEA (80:15:5) for 10 minutes and then washed 3 times with 10ml DMF. The resin can be stored under DMF at room temperature. To load the resin with Ni²⁺-ions, the resin is incubated for 30 minutes with a satured solution of NiCl₂ in DMF. The resin is washed with DMF until no more NiCl₂ can be seen. After 5 additional DMF-washings the following procedure is applied:
- 4x5': DMF/N-Methylimidazol (0.25mol/l)
- 4x15': DMF/N-Methylimidazol (0.25mol/l)
- 1x12h: DMF/N-Methylimidazol (0.25mol/l)
- 2x5': DMF
- 1x1': Isopropanol
- 1x15': Isopropanol
- 1x5': DMF
- 1x30': DMF
- 1x1': Isopropanol
- 1x15': Isopropanol
- 1x5': DMF
- 1x30': DMF
- 1x1': Isopropanol
- 1x15': Isopropanol
- 1x5': DMF
- 1x30': DMF

### Example 10: 5-Amino-1,10-phenantroline / Novasyn TG carboxy-Resin

5g of Novasyn TG Resin are equilibrated with 50ml dry DMF for 30 min. 10mmol DIEA and 10mmol HOBt are added. After 5min. of incubation 2g of 5-amino-1,10-phenanthroline and 10mmol Pybop as well as 10mmol DIEA are added and the mixture vortexed overnight. The next morning the resin is washed 6 times with 50ml DMF each. For loading the resin with Nickel 10 ml of a saturated solution of Nickel chloride in DMF are added and vortexed for 30 min. After this step the supernatant is removed and the resin is washed with batches of 50 ml DMF until no visible colour can be seen in the supernatant. Adsorbed excess of Nickel ions is removed by the following washing procedure:
- 4x5': DMF/N-Methylimidazol (0.25mol/l)
- 4x15': DMF/N-Methylimidazol (0.25mol/l)
- 1x12h: DMF/N-Methylimidazol (0.25mol/l)
- 2x5': DMF
- 1x1': Isopropanol
- 1x15': Isopropanol
- 1x5': DMF
- 1x30': DMF
- 1x1': Isopropanol
- 1x15': Isopropanol
- 1x5': DMF
- 1x30': DMF
- 1x1': Isopropanol
- 1x15': Isopropanol
- 1x5': DMF
- 1x30': DMF

### Example 11: Synthesis of 1,4,7-Triazacyclononane/ 2-Chlortritylchlorid-resin

2.5 g of 2-Chlorotritylchloride-resin (200-400mesh) is suspended in 40 ml dry DMF. 500 mg 1,4,7-Triazacyclononane trihydrochloride and 2 ml diisopropylethylamine is added. The resin is incubated at room temperature for 8 hours. The resin is filtered off and washed with DMF six times. To block unreacted groups on the resin, it is incubated two times with 20ml DCM/MeOH/DIEA (80:15:5) for 30 minutes and then washed 3 times with 20ml DMF. Loading of the resin with Nickel can be done according to example 10. The resin is stored under DMF in the refrigerator.

### Example 12: Loading of tags onto the resins (batch experiments)

1ml of the respective resin is washed 3 times with DMF (peptide synthesis grade). 1mg of the tag is solved in 1ml DMF and 1 drop of DIEA is added. 50µl of this solution are diluted with 950µl water and analyzed by HPLC. The rest of the mixture is added to the washed resin and incubated for 30 minutes. To check the attachment of the tag to the resin 50µl of the solution are diluted with 950µl water and analyzed by HPLC.

### Example 12a: Loading of TAG1 onto Ni-Phenantrolin-2Cl-Trt-Resin

The HPLC of the supernatant liquid shows no more detectable TAG1.

### Example 12b: Loading of TAG3 onto Ni-Phenantrolin-2Cl-Trt-Resin

The HPLC of the supernatant liquid shows no more detectable TAG3. The remaining traces with a similar retention time are minor impurities that did not bind. This example also shows the potential of the method for purification of products from contaminants.

### Example 12c: Loading of TAG4 onto Ni-Phenantrolin-2Cl-Trt-Resin

The HPLC of the supernatant liquid shows no more detectable TAG3.

### Example 12d: Loading of TAG3 onto Ni-Phenantrolin-Novasyn-TG-resin

The HPLC of the supernatant liquid shows no more detectable TAG3.

### Example 13: Elution of TAG3 from the resins (batch experiments)

10ml of the loaded resin are washed 3 times with 20ml of mixture 1 and the resin splitted into 10 portions. The supernatant liquid is removed and the resins incubated for 5 minutes with the following solutions:
- (TAG3_01): 6mg glutathione (oxidized) in 2ml mixture 1 (5mmol/l)
- (TAG3_02): 3mg glutathione (reduced) in 2ml mixture 1 (5mmol/l)
- (TAG3_03): 2ml 1N HCl
- (TAG3_04): 2ml 0.1N HCl
- (TAG3_05): 0.68mg imidazole in 2ml mixture 1(5mmol/l)
- (TAG3_06): 1.36mg imidazole in 2ml mixture 1 (10mmol/l)
- (TAG3_07): 2.72mg imidazole in 2ml mixture 1 (20mmol/l)
- (TAG3_08): 0.82mg N-methylimidazole in 2ml mixture 1 (5mmol/l)
- (TAG3_09): 1.64mg N-methylimidazole in 2ml mixture 1 (10mmol/l)
- (TAG3_10): 3.28mg N-methylimidazole in 2ml mixture 1 (20mmol/l)

For experiments on the elution with higher concentrations of imidazole and N-methylimidazole the experiments are carried out by using the resins from tubes (TAG3-07) and (TAG3_10). These resins are incubated with increasing concentrations of the elution reagents (100mmol, 200mmol, 500mmol), the supernatant liquid is analyzed and the resin is incubated for 5 minutes with the next concentration of elution reagent.

### Results:

Elution of the tags can be easily achieved with hydrochloric acid. Concentrations above 100mmol/l of imidazole or N-methyl-imidazole also elute effectively.

### Example 14: Binding and Elution of TAG3 from Ni-NTA Resin (FPLC-Experiments)

Peptide: TAG 3 (Fmoc-(HiS)₆-OMe, M = 1191.25 g/mol), HPLC-grade
Resin: Ni-NTA SuperFlow (Capacity about 5 µmol/ml)
Column: Biorad 2.0 ml
FPLC-Equipment: Äkta Pharmacia

### a) Elution with N-Methylimidazole

1.1 mg (0.92 µmol) TAG 3 in 0.75 ml Eluent A were injected
Eluent A: 2,2,2-Trifluorethanol/H₂O/Diisopropylethylamin/*N*-Methylimidazol 1:1:+1%:+5mM
Eluent B: TFE/H₂O/DIEA/NMI 1:1:+1%:+500mM
Flow: 1 ml/min
Program: Inject (5 ml A) → Wash (10 ml A) → Gradient: Elution (0 % B → 100 % B for 10 ml) → Hold (5 ml B) → Regeneration (8 ml A)

TAG 3 is bound quantitatively and elutes at N-methylimidazole concentrations of 100-125mM. Such an FPLC-procedure can also be used to determine threshold concentrations, above which the anchoring molecule is effectively competed by the chelating agent. Below the threshold concentration, the anchoring molecule is still bound to the resin. In a batch procedure, dilution of the mixture to a point below a threshold concentration would refix the dissolved anchoring molecule to the resin.

### b) Elution with hydrochloric acid

2.4 mg TAG 3 (2.0 µmol) in 0.75 ml Eluent A are injected in an Äkta FPLC machine. The following program is run:
Eluent A1: TFE/H₂O/DIEA/NMI 1:1:+1%:+5mM
Eluent B: TFE/H₂O/NMI 1:1:+5mM
Eluent A2: TFE/H₂O/HCl 1:1:+10mM
Flow: 0.5-1 ml/min
Program: Inject (2.5 ml A1) → Wash (10 ml A1) → Gradient: Remove DIEA (0 % B → 100 % B for 10 ml) → then "Step-Gradient" to eluent A2 (10mM HCl:
20%, 40%, 60%, 70%)
TAG 3 elutes at 60% eluent A2.

### Example 15: Synthesis Cycle with Fmoc-Gly

### Coupling of TAG3 and Fmoc-Gly on TG-resin

1ml of TG-resin which is loaded with TAG3 is washed 3 times with 5ml DMF. To deprotect the N-term 5ml 20% Piperidin is added and the resin incubated for 30 minutes. The resin is then washed 6 times with DMF and a preactivated solution of 1mmol Fmoc-Gly-OH, 2mmol DIEA, 2mmol HOBt and 2mmmol DIC

in a minimum of DMF is added. After 60 minutes the resin is washed 6 times with 5ml portions of DMF and 6 times with 2ml portions of water. To cleave the peptide from the resin, 1ml 1N HCl is added and the resin incubated for 30 minutes.

Result: The cleaved peptide was analysed by LCMS on a Vydac C18 mass spec column (218MS5415). A gradient from 5 to 95% B over a period of 30 minutes and at a flow rate of 1 ml/min was applied (A=water, 0.1% TFA; B= acetonitrile, 0.085% TFA).

No Educts could be detected. The only peptide present in the mixture showed the expected mass (M=1247, M2+ = 625). Besides HOBt no other significant impurities could be detected.

### Example 16: Synthesis of TAG5-G-A-Fmoc on Ni-NTA-Resin

2.65mg TAG5 were bound in a 1.7ml Omnifit Column on Ni-NTA-resin. The resin was transferred into a microwave reaction vessel and washed 6times with 5ml DMF each. Supernatants were discarded and the resin incubated with 5ml 25% Piperidin in DMF while being exposed to 3x30 sec microwave pulses at 50W each in a Discovery microwave oven (CEM GmbH). The Resin is washed 6times with DMF. A solution of 1mM of the desired amino acid (appropriately protected) in a minimum of solvent (DMF) is prepared. To this solution 2mmol DIEA, 2mmol HOBt and 2mmol DIC are added. After 5min. of incubation time, this solution is added to the deprotected resin and microwaved for 3x30sec with 50W each. After coupling of the last amino acid, the terminal fmoc group is not removed. The resin is washed 5times with water (pH above 7). Finally, 2ml of 1N HCl is added to the resin. The resin is incubated for 30min., the supernatant removed and preserved. The supernatant is directly used for analytical LC/MS. The resin is collected for recirculation. LC/MS reveales the correct mass of the peptide in the only peptide peak detectable. Non-peptidic contaminations were HOBt and HOBt-H2O

### Example 17: Purification of an IL-2 Immunomer

### Purification of IL-2 Immunomer (DIM03A) on Ni-NTA in 2,2,2-Trifluorethanol/H₂O/diisopropylethylamin/N-Methylimidazol

Peptide: As specified in example 19 (M = 6237 g/mol), crude product from resin cleavage;
   resin: Ni-NTA SuperFlow
76.7 mg (12.2 µmol), in 5 ml eluent A, injected in 5 fractions, see documentation in Figures 3a-c.
column: Biorad 2.0 ml
Eluent A: TFE/H₂O/DIEA/NMI 1:1:+1%:+5mM
Eluent B: TFE/H₂O/DIEA/NMI 1:1:+1%:+500mM
Flow: 1 ml/min
Program: inject (5 ml A) → wash (10 ml A) → gradient: elution (0 % B → 100 % B over 10 ml)→ hold (5 ml B) → regenerate (8 ml A)

The desired product elutes in a single peak at about 125mM NMI. The identity is confirmed by analytical LC-MS.

### Example 18: Postsynthetic Modification of a Peptide

Closure of the disulfide bridge in the peptide

H₂N-HHHHGC_{(Acm)}GNGGGC_{(Trt)}GSGN-COOH

Acm and Trt are standard protecting groups for the SH-moiety at the cysteine residues. The peptide was synthesized by routine peptide synthesis and prepurified on an LC/MS System to a purity above 95%.

1ml of Ni-NTA-resin which is loaded with the peptide is washed 3 times with 2ml MeOH. During 2h 50µl iodine solution (0.5mol/l) is added and the mixture incubated for additional 2h at room temperature. The resin is washed 6 times with MeOH, 3 times with water (pH>7) and then incubated with 2ml HCI (1N). The resin is filtered off and the solution analyzed by LCMS. A single product peak is obtained, which shows the correct mass.

### Example 19: Synthesis of an Interleukin-2 Immunomer using refolding steps of the procedure provided here

Chemical Formula of the substance to be synthesized: 
S-S

HHHH-XX-TIVESCNRWITFAQSIISTLT-βAla-G-G-βAla-TKKTQLQLEHLLLDLQMCLNGINN-XX
 X = D-Alanin
βAla = beta-Alanin
other letters = standard amino acid code

### General strategy:

The synthesis is carried out on 2-chlorotrityl chloride resin (200-400 mesh) with a substitution rate of 0.2mmol/g. The first seven amino acids are coupled as a fragment. Attachement of the following amino acids is achieved by single, double or triple coupling with 10fold excess of amino acids and PyBOP/HOBT/DIPEA as coupling additives. N-terminal deprotection of the growing peptide chain is achieved by double treatment with piperidine/DMF (1/3). In difficult cases a third treatment is done with DBU/piperidine/DMF (2/2/96). The number of coupling and deprotection cycles used and is shown in the following scheme (Information on difficult couplings/deprotections gained by monitoring of each elongation by HPLC-MS.)
RES-XXNNIGN-LCMQLDLLLHELQLQTKKTBGGB-TLTSIISQAFTIWRNCSEVITXXHHHH
COUPLING: 22221211111111122222222-223333333333333333333333333
DEPROTECT: 22222222222222322222222-223333333333333333333333333
1,2,3 = number of coupling or deprotection cycles
X = D-Alanine
B = β-Alanine

### Protocol 1: Synthesis of the first peptide fragment

2mmol of the first amino acid and 4mmol DIPEA are dissolved in 10ml dry DCM. This solution is added to 1.0g of dry 2-chlorotrityl chloride resin (200-400 mesh) and the mixture allowed to react for 60 minutes on a vortexer. At the end of the reaction the resin is allowed to react twice with 20ml of DCM/MeOH/DIPEA for 3 minutes, washed twice with 20ml DCM and twice with DMF. The resin is then treated twice with 20ml piperidine/DMF (1:3) for 3/20 minutes and washed 6 times with 20ml DMF. The attachement of amino acids 2-7 is achieved by the following procedure: 5mmol of the amino acid, 7.5mmol HOBT and l0mmol DIPEA are dissolved in 15ml DMF. After 5 minutes 5mmol of PyBOP and l0mmol DIPEA are added and this solution is poured onto the resin. After 60 minutes on a vortexer the resin is washed 6 times with 20ml DMF, treated twice with 20ml piperidine/DMF (1:3) for 20/20 minutes and washed 6 times with 20ml DMF. In case of the N-terminal amino acid the resin is not treated with piperidine/DMF.

### Protocol 2: Cleavage of the peptide fragment

After attachment of the last amino acid the resin is washed 6 times with 20ml DMF, twice with 20ml DCM and then allowed to react with 50ml TFE/DCM (2/8) for 60 minutes. The resin is filtered off, the solvents removed in vacuo and the crude peptide fragment used without further purification.

### Protocol 3: Reattachement of the peptide fragment

1mmol of the fragment and 2mmol DIPEA are dissolved in 50ml dry DCM. This solution is added to 5.0g of dry 2-chlorotrityl chloride resin (200-400 mesh) and the mixture allowed to react for 12 hours on a vortexer. At the end of the reaction the resin is allowed to react twice with 50ml of DCM/MeOH/DIPEA for 3 minutes, washed twice with 50ml DCM and twice with 50ml DMF.

### Protocol 4: Coupling of amino acids 8-57

The dried resin is allowed to swell in 50ml piperidine/DMF (1/3) for 30 minutes, treated with 30ml piperidine/DMF (1/3) for 20 minutes, treated with 30ml of DBU/piperidine/DMF (2/2/96) for 20 minutes and washed 6 times with 30ml DMF. 10mmol of the amino acid, 15mmol HOBT and 20mmol DIPEA are dissolved in 30ml DMF. After 5 minutes 10mmol of PyBOP and 20mmol DIPEA are added and this solution is poured onto the resin. After 60 minutes on a vortexer the resin is washed twice with 30ml DMF and the coupling repeated once or (in difficult cases) twice for 60 minutes. The resin is then washed 6 times with 30ml DMF. The resin can directly be used to couple the next amino acid or - after 2 washings with 30ml DCM - dried in vacuo and stored at-80°C.

### Protocol 5: Cleavage and deprotection

After coupling of the last amino acid the N-terminal protecting group is removed by a double treatment with 30ml piperidine/DMF (1/3) for 20 minutes and treatment with DBU/piperidine/DMF (2/2/96) for 20 minutes. The resin is washed 6 times with 30ml DMF, twice with 30ml DCM and treated with 50ml TFE/DCM (2/8) for 180 minutes. After filtration the solvent is removed in vacuo and the protecting groups removed by treatment with 30ml TFA/TIS/EDT/water (94/1/2.5/2.5) for 180 minutes under an inert atmosphere. This solution is poured into 300ml cold ether, the precipitate dissolved in acetonitrile and the peptide purified by RP-HPLC (Kromasil 100 C4 10µm, 250x4.6mm) and the collected fractions directly used for the refolding procedure.

Alternatively, the IL-2 immunomer raw product can be purified effectively on a metal-affinity column:
IL-2 raw product, *76.7 mg (12.2 µmol),* in about 5 ml Eluent A, injected in 5 rounds (see also Figures 3a-c)
Column: Omnifit *1.7 ml*
Eluent A: TFE/H₂O/DIEA/NMI 1:1:+1%:+5mM
Eluent B: TFE/H₂O/DIEA/NMI 1:1:+1%:+500mM
Flow: 1 ml/min
Program: Inject (5 ml A) → Wash (16.5 ml A) → Gradient: Elute (0 % B → 100 % B über 10 ml) → Hold (5 ml B) → Regenerate (8 ml A)

### Protocol 6: Refolding Procedure

The respective elution fractions were diluted to a final volume of 20ml using exactly the same solvent as being present in the elution fraction. This solution of the purified product was incubated for 30 min. at room temperature with 10ml Ni-NTA Superflow (Qiagen) under slight agitation in a beaker. The Superflow particles were packed into an empty FPLC Column and attached to an FPLC-machine. Using the chromatography programme of this machine, the solvent was exchanged in a 10 min. Gradient by Water, then another 10 min. gradient was used to change the solvent to a mixture of water/trifluorethanol (1/1). During a 24h period, this solvent was oxygenized by bubbling oxygen through the reservoir bottle and using oxygen as atmosphere in the bottle in order to close the disulfide bridge. During oxygenation, a constant flow of 0.1 ml/min was passed overnight along the column, while the eluate was constantly recirculated into the reservoir bottle.

At the end of the 24h period, the final product, being refolded and sealed by disulfide bridging was eluted by the use of Phosphate Buffered Saline (PBS, pH 7,2)containing 150mM imidazole or by using 0.1M Acetate buffer (pH 4.5).

### Example 20: Coupling of (Bis-tert-butoxycarbonylmethyl-amino)-acetic acid [TAG8b] to the N term of a peptide

1 mmol of a peptide with the sequence WETGLRLAPL has been prepared on a 2-chlorotrityl chloride resin (200-400 mesh) following standard procedures as described in Example 19, protocol 1.

In order to couple Tag8b to the N term of the peptide the procedure for coupling amino acids are applied analogously. 5mmol of Tag8b, 7.5mmol HOBT and 10mmol DIPEA are dissolved in 15ml DMF. After 5 minutes 5mmol of PyBOP and 10mmol DIPEA are added and this solution is poured onto the resin. After 60 minutes on a vortexer the resin is washed 6 times with 20ml DMF, twice with 30ml DCM.

Cleavage and deprotection of the peptide is affected following standard procedures: The resin is treated with 50ml TFE/DCM (2/8) for 180 minutes. After filtration the solvent is removed in vacuo and the protecting groups removed by treatment with 30ml TFA/TIS/water (95/2.5/2.5) for 180 minutes under an inert atmosphere. This solution is poured into 300ml cold ether, the precipitate dissolved in acetonitrile and the peptide purified by RP-HPLC (Kromasil 100 C4 10µm, 250x4.6mm).

During the deprotection step the tert.-butyl esters groups of the Tag are cleaved as well to yield the peptide Tag8-WETGLRLAPL.
LC-MS (ESI): (M+H)⁺: 1331

### Example 21: Coupling of 4-(Bis-tert-butoxycarbonylmethylcarbamoyl)-butyric acid [TAG10b] to the N term of a peptide

1 mmol of a peptide with the sequence GDQYIQQAHRSHI has been prepared on a 2-chlorotrityl chloride resin (200-400 mesh) following standard procedures as described in Example 19, protocol 1.

In order to couple Tag10b to the N term of the peptide the procedure for coupling amino acids are applied analogously. 5mmol of Tag10b, 7.5mmol HOBT and 10mmol DIPEA are dissolved in 15ml DMF. After 5 minutes 5mmol of PyBOP and 10mmol DIPEA are added and this solution is poured onto the resin. After 60 minutes on a vortexer the resin is washed 6 times with 20ml DMF, twice with 30ml DCM.

Cleavage and deprotection of the peptide is affected following standard procedures: The resin is treated with 50ml TFE/DCM (2/8) for 180 minutes. After filtration the solvent is removed in vacuo and the protecting groups removed by treatment with 30ml TFA/TIS/water (95/2.5/2.5) for 180 minutes under an inert atmosphere. This solution is poured into 300ml cold ether, the precipitate dissolved in acetonitrile and the peptide purified by RP-HPLC (Kromasil 100 C4 10µm, 250x4.6mm).

During the deprotection step the tert.-butyl esters groups of the Tag are cleaved as well to yield the peptide Tag10-GDQYIQQAHRSHI.
LC-MS (ESI): (M+H)⁺: 1783

## Claims

1. Use of an activated solid phase comprising a solid support, metal chelating ligands covalently bound to the solid support, metal ions Mⁿ⁺ with n = 1 to 3 coordinatively bound to said metal chelating ligands, said activated solid phase providing coordination sites for the coordinative and reversible attachment of an anchoring part of a peptide for solid phase peptide purification.

2. The use of claim 1, wherein the solid support is based on silica, glass or cellulose or a polymer selected from the group consisting of polystyrene resins, melamine resins and polyvinyl alcohols.

3. The use of any of claims 1 or 2, wherein each metal chelating ligand contains at least one nitrogen, oxygen, phosphor or sulfur atom which is able to establish a coordinative ligand-metal bond.

4. The use of any of claims 1 to 3, wherein each metal chelating ligand contains at least one functional group selected from the group consisting of amino, heterocyclic nitrogen, carboxy, hydroxyl and mercapto.

5. The use of any of claims 1 to 4, wherein each metal chelating ligand covalently bound to the solid support contains at least one moiety selected from the group consisting of triphenylphosphine moieties, aminopurine moieties, preferably 6-aminopurine moieties, phthalocyanine moieties, 1,10-phenanthroline moieties, preferably 5-amino-1,10-phenanthroline moieties, terpyridine moieties, preferably 4'-amino-[2,2';6',2"]terpyridine moieties, triazacyclononane moieties, preferably [1,4,7]triazacyclononane moieties and tetraazacyclododecanyl moieties, preferably [1,4,7,10]tetraazacyclododecane moieties.

6. The use of any of claims 1 to 5, wherein the metal Mⁿ⁺ is selected from the group consisting of Mn²⁺, Cu²⁺, Ni²⁺, Co²⁺, Zn²⁺, Mg²⁺, Ca²⁺, Fe2+, Fe3+ and lanthanide ions, particularly preferred Mⁿ⁺ is Cu²⁺, Ni²⁺, Co²⁺, Zn²⁺, Mg²⁺.

7. A process wherein an anchoring part of a peptide is coordinatively bound to coordination sites of an activated solid phase comprising a solid support, metal chelating ligands covalently bound to the solid support, metal ions Mⁿ⁺ with n = 1 to 3 to said metal chelating ligands, is detached from said activated solid phase by addition of a competitive ligand.

8. The process of claim 7, wherein the competitive ligand contains at least one moiety able to chelate metal ions, preferably a nitrogen containing moiety, selected from the group consisting of imidazole, *N*-methylimidazole, aminopurine, phenanthroline, bipyridine, terpyridine, triazacyclononane, tetraazacyclododecane, iminodiacetic acid moieties, nitrilotriacetic acid moieties and ethylendiaminetetraacetic acid moieties.

9. The process of claims 7 or 8, wherein the peptide is a growing peptide, bound via an anchoring part to a metal ion, which is bound to a metal chelating ligand bound to a solid support and subject to peptide elongation procedures.

10. The process of claim 9, wherein mono- or oligomeric amino acids are added at the C- or N-terminus to the growing peptide in a Merrifield-type sequential reaction schedule.

11. The process of any of claims 7 to 10, wherein the anchoring part of the peptide contains at least one metal ion complexing moiety, each said moiety comprising at least one nitrogen, oxygen, phosphor or sulfur containing group which is able to coordinate to the metal ions of the activated solid phase.

12. The process of any of claims 7 to 11, wherein the nitrogen containing group being able to coordinate to the metal ions of the activated solid phase, is selected from the group consisting of amino, hydroxyl, carboxyl, mercapto, imidazolyl, *N*-methylimidazolyl, aminopurinyl moieties, phenanthrolyl moieties, pyridyl moieties, bipyridyl moieties, terpyridinyl moieties, triazacyclononanonyl moieties, tetraazacyclododecanyl moieties, iminodiacetic acid moieties, nitrilotriacetic acid moieties and ethylenediaminetetraacetic acid moieties.

13. The process of any of claims 7 to 12, wherein the anchoring part of the peptide chain is located at the C-terminus and/or in at least one amino acid side chain of the peptide.

14. The process of claim 13, wherein at least one amino acid of the anchoring part at the C-terminus of the peptide is extended by one or more amino acids, which allows detection by detection systems.

15. The process of any of claims 7 to 14, wherein the anchoring part of the peptide is extended at its N-terminus by an amino acid sequence providing a recognition site for a specific protease.

16. The process of any of claims 7 to 15, wherein, after detachment, the peptide is reattached to the activated solid phase by diluting the reaction mixture of the Merrifield-type sequential reaction schedule containing the competitive ligand.

17. A process for purification of, optionally protected, peptides containing an anchoring part, said anchoring part is coordinatively bound to coordination sites of an activated solid phase comprising a solid support, metal chelating ligands covalently bound to the solid support, metal ions Mⁿ⁺ with n = 1 to 3 to said metal chelating ligands, is rinsed, to wash away contaminants such as remnants of protecting groups and scavengers or undesired side products of peptide synthesis.

18. A process for refolding misfolded structures and/or deaggregating intermolecular aggregates of an, optionally protected peptide, wherein the anchoring part of the peptide is coordinatively and reversibly attached to an activated solid phase, and re-establishing a correctly folded peptide structure, comprising the steps of
(a) exposure of the peptide to at least one chaotropic or denaturing agent, and
(b) (b) subsequent exposure to a sequence of solvents to gradually reduce chaotropy and to provide reproducible conditions of refolding and re-establishment of secondary and tertiary structure.

19. The process of claim 18, wherein the secondary and tertiary structure of a peptide is maintained by covalent links between reactive side chains of said peptide by treating the peptide with suitable agents, comprising the formation of said covalent links prior to detachment of the peptide from the activated solid phase.

20. The process of claim 19, wherein the covalent links are disulfide bonds, amide bonds or stable aromatic or aliphatic hydrazones.

21. The process of claims 7, 17, 18 and 19 for the synthesis of a peptide of the sequence
**HHHH-XX-TIVESCNRWITFAQSIISTLT-**β**Ala-G-G-**β**Ala-TKKTQLQLEHLLLDLQMCLNGINN-XX** **(I)**
with, X = d-alanine and βAla = beta-alanine, comprising the formation of a disulfide bond between the cysteine residues, thus forming 
S—S
HHHH-XX-TIVESCNRWITFAQSIISTLT-βAla-G-G-βAla-TKKTQLQLEHLLLDLQMCLNGINN-XX,
 wherein X and βAla are as defined above.

22. A peptide containing an anchoring part preferably consisting of unnatural amino acids for coordinative and reversible attachment of the peptide to the surface of an activated solid phase, which anchoring part is located at the N-or C-terminal and/or in a side chain of the peptide, contains at least one metal ion complexing moiety, each said moiety comprising at least one nitrogen containing group, with the exception of peptides, wherein said metal ion complexing moiety is an amino acid sequence of 2 to 5 histidine residues.

23. The peptide of claim 22, wherein the metal ion complexing groups, preferably nitrogen containing groups, are fixed to the N- or C- terminus or part of the side chain of at least one amino acid and are selected from the group consisting of amino, hydroxyl, carboxyl, mercapto, imidazolyl, *N-*methylimidazolyl, aminopurinyl moieties, phenanthrolyl moieties, pyridyl moieties, bipyridyl moieties, terpyridinyl moieties, triazacyclononanonyl moieties and tetraazacyclododecanyl moieties, or combinations thereof.

24. An anchoring part for coordinative anchoring of a growing peptide chain to an activated solid phase, preferably being a mono- or oligomeric peptide consisting of natural and/or unnatural, preferably of unnatural amino acids.
